# EUROPEAN PATENT APPLICATION

(11) **EP 4 603 023 A1**
(43) Date of publication of application: **20.08.2025**
(21) Application number: 23876424.5
(22) Date of filing: 08.09.2023
(51) Int. Cl.: A61B 8/00, G09G 3/00

(54) **INFORMATION PROMPTING APPARATUS AND SYSTEM, AND ULTRASONIC SCANNING DEVICE**

(30) Priority: 10.10.2022 CN 202211235168
(71) Applicant: Yukun (Beijing) Technology Co., Ltd., Beijing 102200 (CN)
(72) Inventor: QIU, Xiaokang, Beijing 102200 (CN); ZHANG, Qianyi, Beijing 102200 (CN)
(74) Representative: Murgitroyd & Company
(86) International application number: PCT/CN2023/117669
(87) International publication number: WO 2024/078219

(57) **Abstract**

The present application provides an information prompting device and system, and an ultrasonic scanning apparatus. The device includes a display module and a light source module for illuminating a display area of the display module. The display module is configured to display an ultrasonic scanning video frame obtained by scanning in the display area. The light source module is connected to a detection module, and is configured to receive abnormal position information obtained by the detection module detecting the ultrasonic scanning video frame, and illuminate, based on the abnormal position information, an abnormal position in the ultrasonic scanning video frame displayed in the display area as a prompt. The information prompting device of the present application can illuminate, by the light source module, abnormal information detected by the detection module in the display area of the display module. Thus, the light beam emitted by the light source module can be incident on a part of content displayed on the display module, achieving the effect of simultaneously displaying the abnormal information and the display information, with no additional display screen.

## Description

### TECHNICAL FIELD

Some embodiments of the present application relate to, but are not limited to, medical device technologies, and in particular, to an information prompting device and system, and an ultrasonic scanning apparatus.

### BACKGROUND

Ultrasonic scanning is a common imaging method in modem medicine. During ultrasonic scanning, a person usually holds a scanning apparatus to press a target area of the human body and slowly scan it, thereby generating an ultrasonic scanning video image.

With the development of artificial intelligence (AI) medicine, during ultrasonic scanning, a physiological tissue structure in the scanned area can be presented in real time and, in addition, an abnormal phenomenon in the scanned area can be identified though AI technology and a prompt for the identified abnormal phenomenon may be provided, where an ultrasonic scanning video including the identified abnormal phenomenon or an ultrasonic scanning image including the identified abnormal phenomenon may be displayed.

Generally, during ultrasonic scanning, a doctor needs to perform ultrasonic scanning while viewing an ultrasonic scanning video or image obtained by scanning. However, another separate prompt display screen is used to display the AI prompt. Thus, two display screens are used therein to display different contents respectively, and the number of the display screens is more than one.

### SUMMARY

The present application provides an information prompting device, where abnormal information detected by a detection module can be illuminated on the display module by a light source module, thereby achieving the effect of simultaneously displaying the abnormal information and the display information, with no additional display screen.

The following is a summary of the subject matter described in detail herein. This summary is not intended to limit the scope of the claims.

To achieve the above objective:
In a first aspect, the present application provides an information prompting device including a display module and a light source module configured to illuminate a display area of the display module;
wherein the display module is configured to display an ultrasonic scanning video frame obtained by scanning in the display area; and
the light source module is connected to a detection module and configured to: receive abnormal position information obtained by the detection module detecting the ultrasonic scanning video frame; and illuminate, based on the abnormal position information, an abnormal position in the ultrasonic scanning video frame displayed in the display area as a prompt.

Optionally, the light source module includes:
a beam irradiation module and a control module, wherein the beam irradiation module is electrically connected to the control module, and the control module is electrically connected to the detection module; and
the control module is configured to receive the abnormal position information obtained by the detection module detecting the ultrasonic scanning video frame, and control, based on the abnormal position information, the beam irradiation module to project a beam on the ultrasonic scanning video frame displayed in the display area, to illuminate the abnormal position in the ultrasonic scanning video frame displayed in the display area as the prompt.

Optionally, the light source module includes:
a beam irradiation module, a control module and a fluorescent film, wherein the beam irradiation module is electrically connected to the control module, the control module is electrically connected to the detection module, and the fluorescent film is attached in the display area; and
the control module is configured to receive the abnormal position information obtained by the detection module detecting the ultrasonic scanning video frame, and control, based on the abnormal position information, the beam irradiation module to project a beam on the fluorescent film attached in the display area, to enable the fluorescent film to form a light spot in the display area to illuminate the abnormal position in the ultrasonic scanning video frame displayed in the display area as the prompt.

Optionally, the beam irradiation module is a laser.

Optionally, the light source module includes a control module and a plurality of illuminating lamps, and the display area includes a plurality of unit display areas respectively corresponding to the plurality of illuminating lamps; and
the control module is configured to receive the abnormal position information obtained by the detection module detecting the ultrasonic scanning video frame, and control a part of the illuminating lamps corresponding to a part of the unit display areas where the abnormal position information is located to light up, to illuminate the abnormal position in the ultrasonic scanning video frame displayed in the display area as the prompt.

Optionally, the plurality of illuminating lamps are arranged along a first direction of the display area and along a second direction of the display area different from the first direction.

Optionally, the plurality of illuminating lamps constitutes at least one first lamp strip extending along a first direction of the display module and at least one second lamp strip extending along a second direction of the display module, and an included angle of 90 degrees is formed between the first direction and the second direction.

Optionally, the plurality of illuminating lamps constitutes a lamp panel spaced apart from the display module, and the plurality of illuminating lamps respectively correspond to the plurality of unit display areas.

In a second aspect, the present application further provides an ultrasonic scanning system including a detection module, a scanning module and the information prompting device as described in any one of the above,
wherein the scanning module is configured to obtain the ultrasonic scanning video frame by scanning an object to be scanned, and the detection module is configured to obtain the abnormal position information by detecting the ultrasonic scanning video frame;
the information prompting device includes the display module and the light source module configured to illuminate the display area of the display module;
the display module is configured to display the ultrasonic scanning video frame obtained by scanning in the display area; and
the light source module is connected to the detection module, and is configured to receive the abnormal position information obtained by the detection module detecting the ultrasonic scanning video frame, and illuminate, based on the abnormal position information, the abnormal position in the ultrasonic scanning video frame displayed in the display area as the prompt.

In a third aspect, the present application further provides an ultrasonic scanning apparatus including a scanning module and the information prompting device as described in any one of the above,
wherein the scanning module is configured to obtain the ultrasonic scanning video frame by scanning an object to be scanned, and the information prompting device includes the display module and the light source module configured to illuminate the display area of the display module;
the display module is configured to display the ultrasonic scanning video frame obtained by scanning in the display area; and
the light source module is connected to the detection module, and is configured to receive the abnormal position information obtained by the detection module detecting the ultrasonic scanning video frame, and illuminate, based on the abnormal position information, the abnormal position in the ultrasonic scanning video frame displayed in the display area as the prompt.

Other aspects will become apparent upon reading and understanding the accompanying drawings and the detailed description.

In the case of a high voltage (U>20V), the electrostatic protection circuit according to the present invention has a larger leakage current than the general electrostatic protection circuit, so that the accumulated electrostatic charge on the row driving signal line of the array substrate can be released in time, avoiding the phenomenon of cross-line electrostatic protection of the circuit; in the case of a low voltage (U≤20V), the load of the row driving signal line of the array substrate is not affected, improving the reliability of the display panel.

### BRIEF DESCRIPTION OF THE DRAWINGS

In order to explain the technical solutions of the present application more clearly, the drawings required to describe some embodiments will be briefly described below.
FIG. 1 schematically shows a structure of an information prompting device according to some embodiments of the present application.
FIG. 2 schematically shows a structure of an information prompting device according to some embodiments of the present application.
FIG. 3 schematically shows a structure of an information prompting device according to some embodiments of the present application.
FIG. 4 schematically shows a structure of an information prompting device according to some embodiments of the present application.
FIG. 5 schematically shows a structure of an information prompting device according to some embodiments of the present application.
FIG. 6 schematically shows a scenario of an information prompting system according to some embodiments of the present application.
FIG. 7 is a schematic block diagram of an ultrasonic scanning apparatus according to some embodiments of the present application.

### DETAILED DESCRIPTION

The technical solutions in some embodiments of the present application will be clearly and completely described below with reference to the accompanying drawings in some embodiments of the present application. It will be apparent that the described embodiments are merely a part of embodiments of the present application, rather than all embodiments. All other embodiments of the present application that can be obtained based on the described embodiments by those skilled in the art without any inventive effort shall fall within the scope of the present application.

It should be appreciated that, in the description of the present application, the terms "first" and "second" are used merely for descriptive purposes and should not be construed as indicating or implying relative importance or implying the number of the defined technical features. Thus, a feature defined by "first" or "second" may explicitly or implicitly include one or more such features. In the description of this application, "a plurality of" means two or more unless otherwise specifically defined.

In the present application, the term "exemplary" means "as an example, instance or illustration". In the present application, any embodiments described as "exemplary" are not necessarily to be construed as preferred or advantageous over other embodiments. In addition, it should be appreciated that, if an embodiment of the present application involving user information, user data or other relevant data is to be applied in a specific product or technology, a user's permission or consent is required, and the collection, use and/or processing of the relevant data should comply with the relevant laws, regulations and standards of the relevant countries and regions.

In order to enable any person skilled in the art to implement and use this application, the following description is given. In the following description, details are provided for the purposes of explanation. It should be appreciated by a person of ordinary skill in the art that the present application may be implemented without these specific details. In other instances, well-known structures and processes will not be described in detail in order to avoid obscuring the description of the present application with unnecessary details. Accordingly, the present application is not limited to the described embodiments, but shall be accorded the broadest scope based on the principles and features disclosed in the present application.

The present application provides an information prompting device and system, and an ultrasonic scanning apparatus, which will be described in detail below.

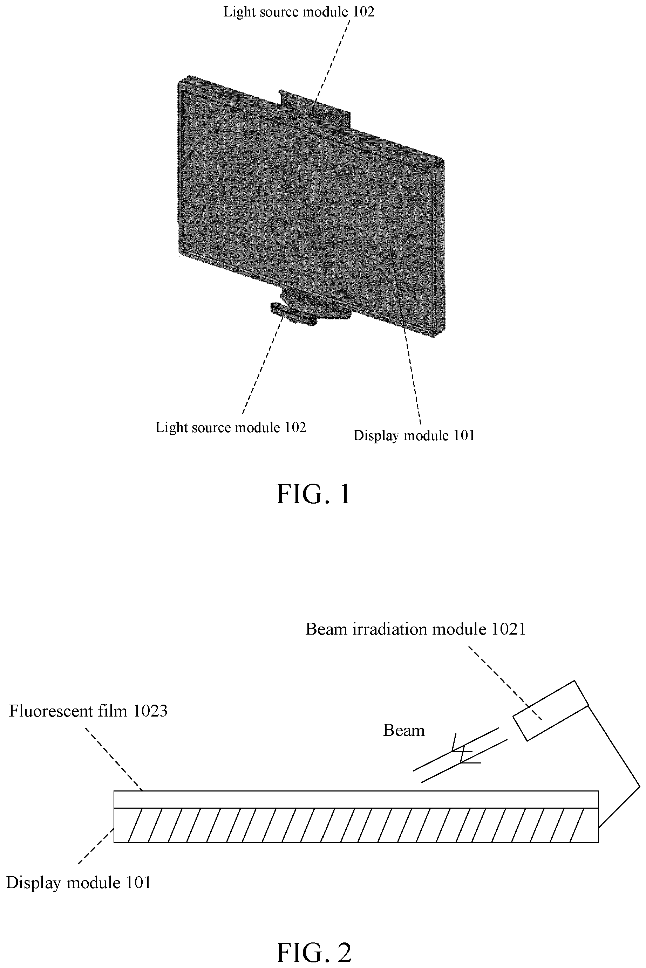

Referring to FIG. 1, FIG. 1 schematically shows a structure of an information prompting device according to some embodiments of the present application. The information prompting device may include the following modules:
a display module 101 and a light source module 102, wherein the source module 102 is configured to illuminate a display area of the display module 101; the display module 101 is configured to display an ultrasonic scanning video frame obtained by scanning in the display area; and the light source module 102 is connected to a detection module 103 and configured to: receive abnormal position information obtained by the detection module 103 detecting the ultrasonic scanning video frame; and illuminate, based on the abnormal position information, an abnormal position in the ultrasonic scanning video frame displayed in the display area as a prompt, where the detection module 103 is not shown in FIG. 1.

In some embodiments of the present application, the display module 101 may be any display device, and the light source module 102 may be any device capable of illuminating as a prompt. A light source in the light source module 102 may be activated to emit light onto the display module 101. The light source module 102 may be mounted on the display module 101 as shown in FIG. 1, and there may be two light source modules 102 respectively mounted above and below the display module 101. When it is necessary to illuminate the display module 102, the light source module 102 may emit light to illuminate a part of the display area of the display module 101 corresponding to the abnormal position information, so that the abnormal position of the content displayed on the display module 101 is highlighted, thereby providing a prompt for abnormality.

The detection module 103 may include a neural network model for detecting abnormal position information. When the information prompting device receives the ultrasonic scanning video, which consists of successive video frames, obtained by a user's real-time scanning operation, the neural network model may detect each video frame of the ultrasonic scanning video to determine whether there is abnormal information therein. In addition, a rotation device may be provided at the connection between the light source module 102 and the display module 101 to adjust the rotation direction of the light source module 102. Thus, the angle of illumination of the display area by the light source module 102 can be changed, and different parts of the display area can be illuminated according to different instructions.

A specific process of detecting one of the video frames may be as follows. When the video frame is input to the neural network model for detection, the feature extraction module of the neural network model may perform feature extraction on the image information of each position in the video frame. Each extracted image feature may correspond to one image position in the video frame and can be further detected by the neural network model to determine whether the extracted image feature is abnormal or not. If it is determined that the extracted image feature is abnormal, the extracted image feature can be determined as abnormal information and then an image position in the video frame corresponding to the abnormal information can be determined based on the image position in the video frame corresponding to the extracted image feature. Since the video frame is displayed on the display module, the display position of the abnormal information on the display module can be determined based on the image position in the video frame corresponding to the abnormal information, as the abnormal position information of the abnormal information.

When the abnormal position information is determined, the light source module 102 can illuminate a part of the display area corresponding to the abnormal position information to improve the brightness of the part of the display area, thereby achieving the effect of prompting. The information prompting device may include a control unit that can control, based on the abnormal position information in the display area, the light source module 102 to illuminate the abnormal position information in the display area.

In addition, in some embodiments of the present application, the light source module 102 may be a laser. The laser light is more concentrated and brighter than the general beam light. When the display module 102 displays the ultrasonic scanning video or image, the display module 102 may generate a certain amount of light. Thus, a better prompt effect can be achieved by using the laser to emit the laser light, which is more concentrated and brighter, to illuminate.

With the information prompting device provided by the present application, while an ultrasonic scanning video or image is displayed on the display module, the detection module can detect the ultrasonic scanning video or image to obtain the abnormal position information and the light source module can illuminate a part of the display area of the display module corresponding to the abnormal position information. Thus, the light beam emitted by the light source module can be incident on the ultrasonic scanning video or image on the display module to provide a prompt for abnormal information on the display module. That is, the ultrasonic scanning video or image and the abnormal information can be simultaneously displayed on one display screen, without an additional display screen.

In order to better implement embodiments of the present application, in an embodiment of the present application, the light source module 102 includes:
a beam irradiation module 1021 and a control module 1022, wherein the beam irradiation module 1021 is electrically connected to the control module 1022, and the control module 1022 is electrically connected to the detection module 103; and the control module 1022 is configured to receive the abnormal position information obtained by the detection module 103 detecting the ultrasonic scanning video frame and control, based on the abnormal position information, the beam irradiator 1021 to project a beam on the ultrasonic scanning video frame displayed in the display area, so as to illuminate an abnormal position in the ultrasonic scanning video frame displayed in the display area as a prompt.

The above embodiments provide a solution where the information prompting device is equipped with the control unit, and another solution where the light source module 102 is equipped with the control module 1022. The control module 1022 may be any integrated control device, such as a single-chip microcomputer, a central processing unit (CPU), or the like.

The detection module 103 may transmit the obtained detection result to the control module 1022, and the control module 1022 generates a control signal based on the detection result to control the beam illumination module 1021 to illuminate the corresponding display area. Specifically, upon obtaining the detection result from the detection module 103, the control module 1022 can determine the specific position of the abnormal information in the ultrasonic scanning video frame or the ultrasonic scanning image. Since the ultrasonic scanning video frame or the ultrasonic scanning image is displayed on the display module 101, the specific display position of the abnormal information in the display area can be determined. Thus, the control module 1022 can control the beam irradiation module 1021 to irradiate a beam onto the corresponding display area, thereby achieving the effect of improving the brightness in the corresponding display area as a prompt.

In addition, in some embodiments of the present application, the beam irradiation module 1021 may be any module capable of emitting a light beam. Compared with a general light source module, the beam irradiation module 1021 can emit more concentrated light and thus can better improve brightness at a given location, thereby facilitating observation by a viewer.

In order to better implement embodiments of the present application, in an embodiment of the present application, the light source module 102 includes:
a beam irradiation module 1021, a control module 1022 and a fluorescent film 1023, wherein the beam irradiation module 1021 is electrically connected to the control module 1022 which is electrically connected to the detection module 103, and the fluorescent film 1023 is attached in the display area; and the control module 1022 is configured to receive the abnormal position information obtained by the detection module 103 detecting the ultrasonic scanning video frame, and control, based on the abnormal position information, the beam irradiation module 1021 to project a beam on the fluorescent film attached in the display area, so that the fluorescent film 1023 forms a light spot in the display area to illuminate the abnormal position in the ultrasonic scanning video frame displayed in the display area as a prompt.

In order to make the prompt through illumination of the display area more easier for a viewer to observe, in the present embodiment, a fluorescent layer may be attached to the surface of the display module 101, as shown in FIG. 2. The fluorescent layer may be the fluorescent film 1023. When the fluorescent film 1023 is illuminated, a light spot may be formed in the illuminated area to provide a prompt. Therein, the control module 1022 is not shown in FIG. 2.

Specifically, in practice, the light irradiated by the beam irradiation module 1021 may not only increase the brightness of the end point illuminated by the light but also itself illuminate the propagation path thereof. With the structure as shown in FIG. 1, the light may be emitted from above the display module 101 or below the display module 101 and illuminate the corresponding display area. However, the light may pass over the display area before reaching the corresponding display area. If there is no abnormality in the space where the light passes, the propagation path illuminated by the light itself may easily result in erroneous observation by a viewer. Therefore, if a transparent fluorescent film 1023 is attached to the surface layer in the display area and the beam irradiation module 1021 is configured to emit invisible light, the invisible light may not interfere with the viewer's observation while passing over the display area. In this case, when the beam irradiation module 1021 illuminates the fluorescent film 1023 in the corresponding display area, the fluorescent film 1023 may form a light spot, as a prompt to the viewer. The material of the fluorescent film may correspond to the wavelength of the invisible light, so that the fluorescent film can convert the wavelength of the invisible light incident thereon into the wavelength of the visible light (refer to, for example, ultraviolet rays, and modes and materials capable of converting the ultraviolet rays into the visible light). In some embodiments of the present application, the beam irradiation module 1021 may be a laser capable of emitting invisible light or a beam irradiation device capable of emitting light with relatively low brightness.

In order to better implement embodiments of the present application, in an embodiment of the present application, the light source module 102 includes a control module 1022 and a plurality of illuminating lamps. The display area includes a plurality of unit display areas respectively corresponding to the plurality of illuminating lamps. The control module is configured to receive the abnormal position information obtained by the detection module 103 detecting the ultrasonic scanning video frame, and control a part of the illuminating lamps corresponding to a part of the unit display areas where the abnormal position information is located to light up, so as to illuminate an abnormal position in the ultrasonic scanning video frame displayed in the display area as a prompt.

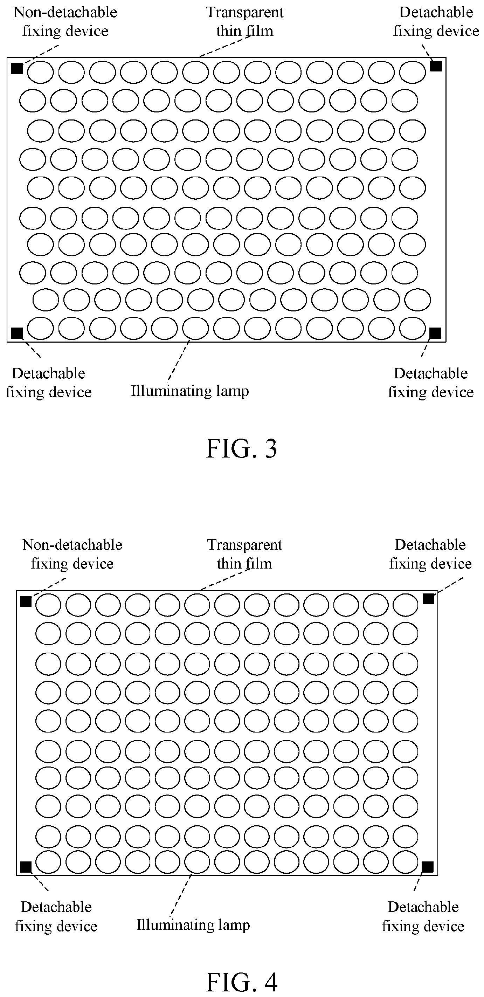

In some embodiments of the present application, a solution of the light source module 102 including a plurality of illuminating lamps is provided. As shown in FIG. 3, the plurality of illuminating lamps may be attached one by one to a transparent thin film which may cover the display area of the display module 101. The transparent thin film has a shape and a size matching those of the display module 101, thereby achieving one-to-one correspondence between the illuminating lamps and the unit display areas. Four fixing devices may be installed respectively at four corners of a frame outside the display area of the display module 101, and four counterpart fixing devices may be installed respectively at four corresponding positions of the transparent thin film. One of the four fixing devices of the transparent thin film is usually non-detachable from one of the fixing devices on the display module 101 and can function to electrically connect the control module 102 with the illuminating lamps. The remaining three fixing devices of the transparent thin film can be detached from the display module 101, so that the viewer can remove the transparent thin film from the display area in order to observe the display area.

Upon obtaining the abnormal position information, the control module 1022 can control a part of the illuminating lamps corresponding to a part of the display area corresponding to the abnormal position information to emit light. Herein, the position of the transparent thin film is the same as the position of the fluorescent film relative to the display module 101. Specifically, upon obtaining the detection result from the detection module 103, the control module 1022 may determine the specific position of the abnormal information in the ultrasonic scanning video frame or the ultrasonic scanning image. Since the ultrasonic scanning video frame or the ultrasonic scanning image is displayed on the display module 101, the specific display position of the abnormal information in the display area may be determined. Thus, the control module 1022 can control a part of the plurality of illuminating lamps corresponding to the display position to emit light, thereby achieving the effect of improving the brightness in the corresponding display area as a prompt.

In order to better implement embodiments of the present application, in an embodiment of the present application, the plurality of illuminating lamps are arranged in a first direction of the display area and in a second direction of the display area different from the first direction.

The above-described embodiment provides a solution in which a plurality of illuminating lamps are attached to a transparent thin film covering the display module 101. Attachment of the illuminating lamps to the transparent thin film in a regular arrangement, whether manually or by machine, can facilitate manufacturing of the transparent thin film with the illuminating lamps attached to. Therefore, in this embodiment, for the sake of making the manufacture of the film including the illuminating lamps easier, the illuminating lamps are arranged in order. As shown in FIG. 4, the illuminating lamps may be arranged in the first direction and in the second direction, where the first direction may be a vertical direction and the second direction may be a horizontal direction. That is, the plurality of illuminating lamps may be arranged in an array. Specifically, upon obtaining the detection result from the detection module 103, the control module 1022 may determine the specific position of the abnormal information in the ultrasonic scanning video frame or the ultrasonic scanning image. Since the ultrasonic scanning video frame or the ultrasonic scanning image is displayed on the display module 101, the specific display position of the abnormal information in the display area may be determined. Thus, the control module 1022 can control a part of the plurality of illuminating lamps corresponding to the display position to emit light, thereby achieving the effect of improving the brightness in the corresponding display area as a prompt.

In order to better implement embodiments of the present application, in an embodiment of the present application, a plurality of illuminating lamps constitute at least one first lamp strip and at least one second lamp strip. The first lamp strip extends in a first direction of the display module, the second lamp strip extends in a second direction of the display module, and the included angle between the first direction and the second direction is 90 degrees.

In the above-described embodiments, a solution in which a plurality of illuminating lamps is attached to a transparent thin film is provided. However, there is a risk that the transparent thin film covering the display module 101 will fall off. If the transparent thin film falls off, the information prompting device will not achieve the effect of information prompting. Therefore, some embodiments of the present application provide a solution of installing the illuminating lamps on the display module 101.

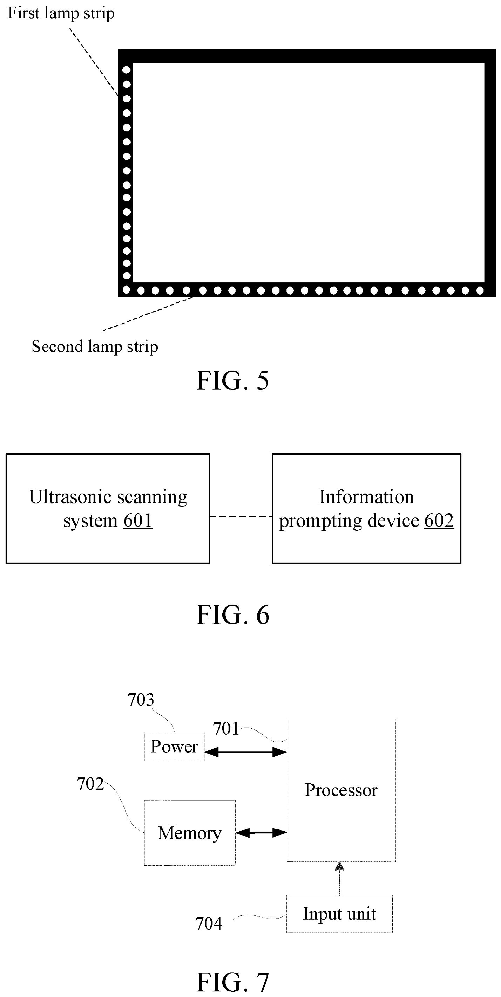

In addition, in order to enable embodiments of the present application to be implemented, in some embodiments, the display module 101 may be a display device including a frame. The illuminating lamps may be arranged on the frame of the display device, as shown in FIG. 5. However, in this case, the illuminating lamps fail to cover the display area of the display module 101. To achieve the effect of prompting, the plurality of illuminating lamps may be regarded as a coordinate system, and the display area may be regarded as a quadrant of the coordinate system. When it is determined that there is abnormal information in the display area, the coordinates in the display area corresponding to the abnormal information may be determined. Based on the determined coordinates, the illuminating lamps on the frame of the display module 101 corresponding to the determined coordinates can be controlled to light up as a prompt. Thus, a viewer may regard the lighted-up illuminating lamps on the frame of the display module 101 as specific coordinates in the display area, and focus observations on the display area corresponding to the coordinates indicated by the lighted-up illuminating lamps. In this way, a prompt for the position of abnormal information in the display area can also be provided. For example, the plurality of illuminating lamps may be arranged respectively on first and second side bars of a rectangular frame of the display module 101. If the fifth illuminating lamp on the first side bar and the tenth illuminating lamp on the second side bar are lighted-up, the viewer can regard a position in the display area corresponding to the coordinates indicated by the lighted-up fifth illuminating lamp on the first side bar and the lighted-up tenth illuminating lamp on the second side bar as a position of the abnormal information. Although this embodiment cannot prompt a viewer by directly increasing the brightness at a corresponding display position in the display area and thus the effect of prompting is reduced to a certain extent, the problem of failing to prompt due to the transparent thin film with the illuminating lamps attached to falling off can be avoided.

In order to better implement embodiments of the present application, in an embodiment of the present application, a plurality of illuminating lamps constitute a lamp panel spaced apart from the display module 101, and the illuminating lamps respectively correspond to the unit display areas.

The above-described embodiments provide a solution in which a plurality of illuminating lamps are attached to a transparent thin film and then the display module 101 is covered by the transparent thin film. However, the transparent thin film is of a soft material and thus may deform. When the transparent thin film deforms, the illuminating lamps attached thereto may be displaced accordingly and the displaced illuminating lamp will not correspond to the original predetermined display area any more, so that the effect of prompting may be reduced. In contrast, in some embodiments, the transparent thin film may be replaced with a transparent plate and a plurality of illuminating lamps are attached to the transparent plate. The transparent plate is of a hard material and thus may not deform, so that the illuminating lamps attached to the transparent plate may not be displaced, which may avoid misalignment between the illuminating lamp and the display area corresponding thereto. It should be noted that, in the present embodiment, only the transparent thin film of the above embodiments to which the illuminating lamps are to be attached is replaced with the transparent plate, the position of the transparent plate relative to the display module 101 remains unchanged, and the transparent plate has a size and a shape matching those of the display module 101. For example, if the display module 101 is a rectangular display, the size of the transparent plate is the same as the size of the rectangular displayer.

In addition, the transparent plate may be attached to the display module 101 in the same manner as the manner in which the transparent thin film is attached to the display module 101 in the above-described embodiments. When a viewer needs to observe the content displayed on the display module 101, the three fixing devices on the transparent plate can be detached from the display module 101 and the remaining one fixing device are not detached therefrom, so that the viewer can observe the content displayed on the display module 101.

Further, the present application provides an ultrasonic scanning system including a detection module, a scanning module and the information prompting device according to any of the above embodiments. FIG. 6 schematically shows a scenario of an ultrasonic scanning system according to some embodiments of the present application. As shown in FIG. 6, the ultrasonic scanning system may include an ultrasonic scanning system 601 and the information prompting device 602. The ultrasonic scanning system 601 may transmit ultrasonic scanning data to the information prompting device 602, so that the information prompting device 602 could perform the prompting operation as described in the present application.

In some embodiments of the present application, the ultrasonic scanning system 601 may communicate with the information prompting device 602 in any communication means, including, but not limited to, mobile communications based on 3rd Generation Partnership Project (3GPP), Long Term Evolution (LTE) or Worldwide Interoperability for Microwave Access (WiMAX), computer network communications based on TCP/IP Protocol Suite (TCP/IP) or User Datagram Protocol (UDP), and the like. Of course, the information prompting device 602 may be installed in the ultrasonic scanning system 601.

It should be noted that the scenario of the ultrasonic scanning system as shown in FIG. 6 is merely an example. The ultrasonic scanning system and the scenario described in some embodiments of the present application are used to more clearly describe the technical solutions according to the embodiments of the present application, and are not intended to limit the technical solutions according to the embodiments of the present application. It may be appreciated by a person of ordinary skill in the art that, with the evolution of ultrasonic scanning systems and the emergence of new service scenarios, the technical solutions according to the embodiments of the present application are also applicable to similar technical problems.

Further, the present application provides an ultrasonic scanning apparatus including a scanning module and the information prompting device according to any of the above embodiments.

That is, the ultrasonic scanning apparatus may incorporate any information prompting device according to some embodiments of the present application. FIG. 7 is a schematic block diagram of the ultrasonic scanning apparatus according to some embodiments of the present application.

Specifically, the ultrasonic scanning apparatus may include components such as a processor 701 with one or more processing cores, a memory 702 with one or more computer-readable storage media, a power supply 703, and an input unit 704. It will be appreciated by those skilled in the art that the ultrasonic scanning apparatus is not limited to the structure as shown in FIG. 7, but may include more or less components than those shown, or certain components may be combined therein, or the components may be in an arrangement different from that shown.

The processor 701 is a control center of the ultrasonic scanning apparatus and connected to various other parts of the ultrasonic scanning apparatus through various interfaces and lines. The processor 701 can perform various functions of the ultrasonic scanning apparatus and process data by running or executing software programs and/or modules stored in the memory 702 and invoking data stored in the memory 702.

Optionally, the processor 701 may include one or more processing cores. The processor 701 may be a Central Processing Unit (CPU), or may be another general-purpose processor, a Digital Signal Processor (DSP), an Application Specific Integrated Circuit (ASIC), a Field Programmable Gate Array (FPGA) or another programmable logic device, a discrete gate or a transistor logic device, a discrete hardware component, or the like. The general-purpose processor may be a microprocessor, or the processor may be any conventional processor or the like.

Preferably, an application processor for mainly processing an operating system, a user interface, an application program and the like and a modem processor for mainly processing wireless communication may be integrated into the processor 701. It will be appreciated that the modem processor may not be integrated into the processor 701.

The memory 702 may be configured to store software programs and modules, and the processor 701 may perform various functional applications and data processing by running the software programs and modules stored in the memory 702. The memory 702 may mainly have a program storage area where an operating system, an application program required for at least one function, and the like may be stored, and a data storage area where data created during use of the ultrasonic scanning apparatus, and the like may be stored .

In addition, the memory 702 may include a high-speed random access memory, and may also include a non-volatile memory, such as at least one magnetic disk storage device, a flash memory device, or another non-volatile solid state storage device. Accordingly, the memory 702 may also include a memory controller to enable the processor 701 to access the memory 702.

The ultrasonic scanning apparatus further includes a power supply 703 for supplying power to various components. Preferably, the power supply 703 may be logically connected to the processor 701 through a power management system, thereby implementing functions, such as charging management, discharging management and power consumption management, by the power management system. The power supply 703 may further include one or more direct current (DC) or alternating current (AC) power supplies, a recharging system, a power failure detection circuit, a power converter or inverter, a power status indicator, or any other component.

The ultrasonic scanning apparatus may further include an input unit 704 that may be configured to receive input number or character information and to generate a keyboard, mouse, joystick, optical or trackball input signal related to user's setting or functional control.

Although not shown, the ultrasonic scanning apparatus may further include a display unit or the like, and details are not described herein. Specifically, in the ultrasonic scanning apparatus according to some embodiments, the processor 701 may load the executable files corresponding to the processes of one or more application programs into the memory 702 according to the related instructions, and the processor 701 may run the application programs stored in the memory 702, thereby realizing an information prompting device connected with the ultrasonic scanning apparatus. For example, the information prompting device may include:
a display module and a light source module, wherein the light source module is configured to illuminate a display area of the display module;
the display module is configured to display an ultrasonic scanning video frame obtained by scanning in the display area;
the light source module is connected to a detection module, and is configured to receive abnormal position information obtained by the detection module detecting the ultrasonic scanning video frame, and illuminate, based on the abnormal position information, an abnormal position in the ultrasonic scanning video frame displayed in the display area as a prompt.

In the above embodiments, the description of each embodiment has its own emphasis. For parts not described in detail in a certain embodiment, reference may be made to the above detailed description for other embodiments, and details are not described herein again.

In practice, the above units or structures may be implemented as independent entities or may be implemented, in any combination thereof, as the same or several entities. For specific implementations of the above units or structures, reference may be made to the above device embodiments, and details are not described herein.

For specific implementations of the above operations, reference may be made to the previous embodiments, and details are not described herein.

The information prompting device and apparatus according to some embodiments of the present application have been described in detail above. Herein, specific examples are used to explain the principle and implementation of the present application. The above description of the embodiments is intended only to help understand the device and the core idea of the present application. Moreover, various modifications of the specific implementation and application scope may occur to those skilled in the art based on the idea of the present application. In summary, the content of the description should not be construed as limitation of the present application.

## Claims

1. An information prompting device, comprising a display module and a light source module configured to illuminate a display area of the display module,
wherein the display module is configured to display an ultrasonic scanning video frame obtained by scanning in the display area; and
the light source module is connected to a detection module, and is configured to receive abnormal position information obtained by the detection module detecting the ultrasonic scanning video frame, and illuminate, based on the abnormal position information, an abnormal position in the ultrasonic scanning video frame displayed in the display area as a prompt.

2. The information prompting device according to claim 1, wherein the light source module comprises:
a beam irradiation module and a control module, wherein the beam irradiation module is electrically connected to the control module, and the control module is electrically connected to the detection module; and
the control module is configured to receive the abnormal position information obtained by the detection module detecting the ultrasonic scanning video frame, and control, based on the abnormal position information, the beam irradiation module to project a beam on the ultrasonic scanning video frame displayed in the display area, to illuminate the abnormal position in the ultrasonic scanning video frame displayed in the display area as the prompt.

3. The information prompting device according to claim 1, wherein the light source module comprises:
a beam irradiation module, a control module and a fluorescent film, wherein the beam irradiation module is electrically connected to the control module, the control module is electrically connected to the detection module, and the fluorescent film is attached in the display area; and
the control module is configured to receive the abnormal position information obtained by the detection module detecting the ultrasonic scanning video frame, and control, based on the abnormal position information, the beam irradiation module to project a beam on the fluorescent film attached in the display area, to enable the fluorescent film to form a light spot in the display area to illuminate the abnormal position in the ultrasonic scanning video frame displayed in the display area as the prompt.

4. The information prompting device according to claim 2 or 3, wherein the beam irradiation module is a laser.

5. The information prompting device according to claim 1, wherein the light source module comprises a control module and a plurality of illuminating lamps, and the display area comprises a plurality of unit display areas respectively corresponding to the plurality of illuminating lamps; and
the control module is configured to receive the abnormal position information obtained by the detection module detecting the ultrasonic scanning video frame, and control a part of the illuminating lamps corresponding to a part of the unit display areas where the abnormal position information is located to light up, to illuminate the abnormal position in the ultrasonic scanning video frame displayed in the display area as the prompt.

6. The information prompting device according to claim 5, wherein the plurality of illuminating lamps are arranged along a first direction of the display area and along a second direction of the display area different from the first direction.

7. The information prompting device according to claim 5, wherein the plurality of illuminating lamps constitute at least one first lamp strip extending along a first direction of the display module and at least one second lamp strip extending along a second direction of the display module, and an included angle of 90 degrees is formed between the first direction and the second direction.

8. The information prompting device according to claim 5, wherein the plurality of illuminating lamps constitute a lamp panel spaced apart from the display module, and the plurality of illuminating lamps respectively correspond to the plurality of unit display areas.

9. An ultrasonic scanning system, comprising a detection module, a scanning module and the information prompting device according to any of claims 1-8,
wherein the scanning module is configured to obtain the ultrasonic scanning video frame by scanning an object to be scanned, and the detection module is configured to obtain the abnormal position information by detecting the ultrasonic scanning video frame;
the information prompting device comprises the display module and the light source module configured to illuminate the display area of the display module;
the display module is configured to display the ultrasonic scanning video frame obtained by scanning in the display area; and
the light source module is connected to the detection module, and is configured to receive the abnormal position information obtained by the detection module detecting the ultrasonic scanning video frame, and illuminate, based on the abnormal position information, the abnormal position in the ultrasonic scanning video frame displayed in the display area as the prompt.

10. An ultrasonic scanning apparatus, comprising a scanning module and the information prompting device according to any of claims 1-8,
wherein the scanning module is configured to obtain the ultrasonic scanning video frame by scanning an object to be scanned, and the information prompting device comprises the display module and the light source module configured to illuminate the display area of the display module;
the display module is configured to display the ultrasonic scanning video frame obtained by scanning in the display area; and
the light source module is connected to the detection module, and is configured to receive the abnormal position information obtained by the detection module detecting the ultrasonic scanning video frame, and illuminate, based on the abnormal position information, the abnormal position in the ultrasonic scanning video frame displayed in the display area as the prompt.
